# FASCICULE DE BREVET EUROPEEN

(11) **EP 2 821 034 B1**
(45) Date de publication et mention de la délivrance du brevet: **12.10.2016**
(21) Numéro de dépôt: 14175542.1
(22) Date de dépôt: 03.07.2014
(51) Int. Cl.: A61F 5/01, A47C 31/12, A61G 5/00, A61F 5/02, A61G 5/12

(54) **Corset orthopédique évolutif**
Erweiterbares orthopädisches Korsett
Adaptive orthopaedic corset

(30) Priorité: 04.07.2013 FR 1356575
(43) Date de publication de la demande: 07.01.2015
(73) Titulaire: Orthotech, 94100 Saint Maur des Fosses (FR)
(72) Inventeur: Belloch, Jean-François, 94100 Saint Maur des Fossés (FR)
(74) Mandataire: Ipside

(56) Documents cités:
- DE-A1- 19 520 585
- US-A- 4 647 066
- US-A- 5 161 522
- US-A- 6 032 975
- US-A1- 2004 084 941
- US-A1- 2006 192 362

## Description

### Domaine de l'invention

L'invention concerne un corset orthopédique évolutif fabriqué sur mesures ou sur moulage pour soutenir et maintenir un patient dans une position choisie. Ce corset orthopédique peut s'adapter aux modifications et évolutions du patient, tant au niveau de sa morphologie que de sa position dans le corset ou de tout autre de ses besoins spécifiques. Ce corset orthopédique est adapté pour positionner et maintenir différents segments corporels, tels que les cuisses, les hanches et/ou le tronc de manière à maintenir le patient dans une position assise. Il peut être adapté également pour maintenir, en position droite, rectiligne, tout ou partie de ses segments corporels, tels que les pieds, les jambes, le bassin, le tronc, etc.

L'invention trouve des applications dans le domaine de l'orthopédie, et en particulier du maintien orthopédique, pour maintenir des patients dont un ou plusieurs segments corporels sont défaillants.

### Etat de la technique

L'utilisation d'un corset orthopédique par un patient est préconisée dans le cas où un ou plusieurs segments corporels d'un patient sont défaillants empêchant le patient de soutenir et maintenir son tronc dans une position choisie. Par exemple, un corset siège est utilisé pour le maintien en position assise d'une personne présentant une déficience de la statique, d'origine neurologique, musculaire, génétique, ou de toute autre pathologie invalidante. Le corset siège permet alors une stabilité en position assise et un soutien du rachis. Il doit également prévenir les risques de déformations orthopédiques, telles que les déformations articulaires du rachis, du bassin et des hanches.

Classiquement, les corsets orthopédiques, et en particulier les corsets sièges, sont fabriqués en moulant successivement, sur un moule représentant la forme du patient dans la position souhaitée, une mousse de confort en polyéthylène thermo-formable, puis une coque en plastique rigide en polyéthylène thermo-formable également. La coque obtenue après démoulage est monolithique.

D'autres corsets orthopédiques sont réalisés en injectant une mousse souple expansée en polyuréthane entre un moule représentant la forme du patient dans la position souhaitée et une coque rigide en polyéthylène thermoformée au préalable sur le moule du patient.

Dans ces deux cas, le corset orthopédique obtenu est figé. Il ne peut pas être adapté aux besoins spécifiques du patient si ces besoins ou sa morphologie changent entre le moment où le moule sur le patient est réalisé et la livraison du corset orthopédique. De même, un corset orthopédique classique ne peut être adapté lorsque des modifications ont lieu, par exemple suite à des analyses de besoins différentiels du patient, ou lorsque la morphologie du patient se modifie, par exemple dans le cas d'un enfant. Il est alors nécessaire de procéder à la réalisation d'un nouveau corset complet.

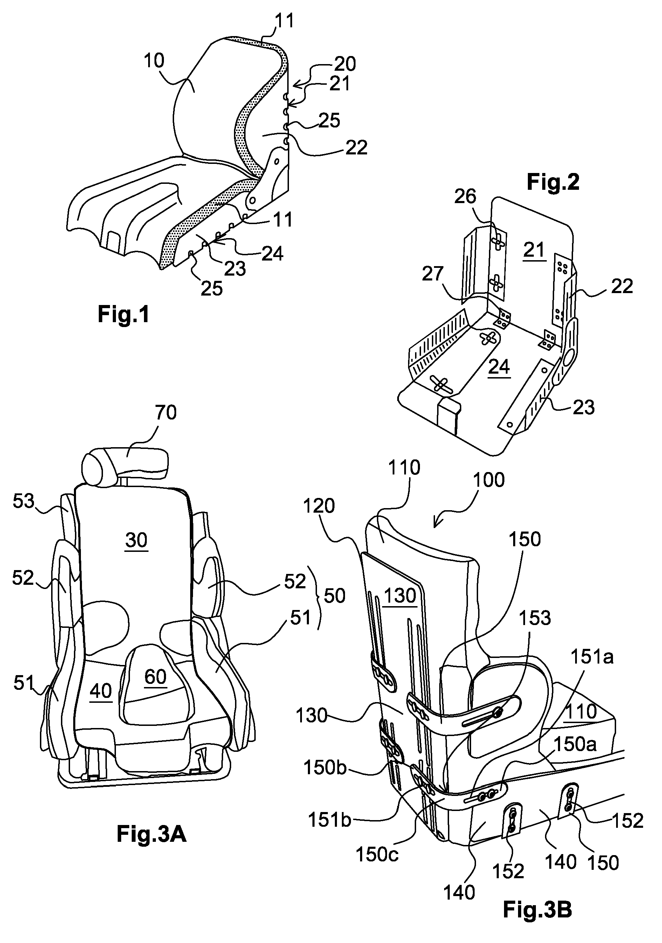

Pour résoudre ce problème, un procédé a été mis au point qui consiste à réaliser un corset interne en mousse indépendant d'un corset externe en plastique thermoformé. Un exemple d'un tel corset siège est représenté sur les figures 1 et 2. Ces figures montrent un corset interne 10, réalisé dans un bloc de mousse de confort 11 creusé strictement à la forme ou aux mesures du patient. La mousse de confort utilisée peut être plus ou moins souple en fonction de la destination finale du corset et du segment corporel à soutenir. Ce procédé consiste, dans un premier temps, soit à réaliser un moulage de la partie à soutenir du patient, à l'aide d'un sac à billes de positionnement d'assise et/ou par un relevé corporel en 3D du corps du patient, pour obtenir un modèle, soit à réaliser des mesures qui servent à configurer un modèle numérique paramétrable. Une fois le modèle achevé, la forme du patient est creusée dans le bloc de mousse. Une coque externe 20, réalisée en plastique thermoformé, est ensuite façonnée et ajustée sur la coque interne 10 en mousse. Cette coque externe 20 comporte un dossier 21 et une assise 24 assemblés l'un avec l'autre au moyen de charnières 27. La coque externe 20 comporte également des renforts latéraux supérieurs 22 et inférieurs 23. Les renforts latéraux 22 et 23 sont montés de manière amovible respectivement sur le dossier 21 et sur l'assise 24 au moyen, par exemple, de vis 26. Ces renforts latéraux étant amovibles, ils peuvent être retirés et remplacés par d'autres plus larges, plus étroits ou angulairement
différents. Ils peuvent aussi être conservés et montés sur un dossier et/ou une assise de dimensions différentes.

Ces dispositions s'inspirent en partie des sièges ajustables bien connus de l'art antérieur tels que divulgués dans la demande de brevet américain N° US 2004/0064941A1 et plus particulièrement des sièges orthopédiques ajustables qui comportent des supports pour la tête, des supports latéraux, des lamelles des fixations et un dossier inclinable tels que présentés dans les brevets américains N° US 4647066 et US 5161522.

Une alternative pour maintenir le patient sur un siège orthopédique consiste à utiliser des coussins pneumatiques disposés sur une chaise rigide en renfort latéral et au dos du patient tel que présenté dans la demande de brevet américain N° US 2006/0192362A1.

Il est connu de l'art antérieur des sièges orthopédiques tels celui défini dans le préambule de la revendication 1, pouvant être modulaires sur lesquels des éléments comme le support pour la tête, les supports latéraux peuvent être retirés et remplacés comme présenté dans le brevet américain N° US 6032975, les éléments peuvent être disposés le long d'un axe central ou colonne centrale tel que présenté dans la demande de brevet allemand N° DE19520585A1.

Toutefois, l'évolution de ce corset est limitée au changement d'éléments du corset externe. Tous les éléments du corset externe étant en plastique thermoformé, ils ont chacun une forme préformée qui ne peut être modifiée. Seuls des éléments peuvent être retirés et remplacés par d'autres de formes ou de dimensions mieux adaptées.

### Exposé de l'invention

L'invention a justement pour but de remédier aux inconvénients des techniques exposées précédemment. A cette fin, l'invention propose un corset orthopédique évolutif comportant un corset interne en mousse et un corset externe formé d'un bâti et de renforts latéraux montés sur des lamelles de fixation, elles-mêmes fixées sur le bâti. Les lamelles de fixation comportent chacune au moins une perforation longitudinale offrant une pluralité de positionnements par rapport aux renforts latéraux et/ou au bâti. Les renforts latéraux n'étant pas directement assemblés avec le bâti, ils peuvent être déplacés par rapport audit bâti dans toutes sortes de configurations, ce qui offre une large panoplie d'évolutions possibles au corset.

De façon plus précise, l'invention concerne un corset orthopédique pour le soutien et le maintien dans une position donnée d'au moins un segment corporel d'un patient, ledit corset orthopédique comportant
- une coque interne en mousse destinée à épouser des contours morphologiques du au moins un segment corporel du patient et comportant au moins un bloc de mousse creusé en fonction des contours morphologiques dudit segment corporel du patient à soutenir, et
- une coque externe à l'intérieur de laquelle est montée la coque interne en mousse et formant une structure rigide de renfort,
- un bâti formant un dossier et une assise, muni de perforations linéaires,
- des renforts latéraux amovibles, et
- des lamelles de fixation perforées aptes à être fixées sur les renforts latéraux, d'une part, et sur le bâti, d'autre part, au moyen d'éléments de fixation introduits dans les perforations linéaires du bâti et les perforations des lamelles de fixation.

Ce corset se caractérise par le fait que le bâti est formé :
- soit par une unique plaque métallique pliée pour former, d'une part, l'assise et, d'autre part, le dossier de la coque externe,
- soit par un premier cadre formant l'assise de la coque externe, et
   par un second cadre formant le dossier de ladite coque externe, une plaque intermédiaire coudée étant fixée sur le premier et sur le second cadres et fermant lesdits premier et second cadres, chaque cadre étant formé d'au moins trois lames perforées fixées les unes aux autres et/ou à la plaque intermédiaire coudée et aptes chacune à recevoir les éléments de fixation des lamelles de fixation.

Ce corset présente l'avantage d'être totalement modulable et évolutif, les renforts latéraux pouvant prendre toutes sortes de positions par rapport au bâti.

Ce corset orthopédique peut comporter une ou plusieurs des caractéristiques suivantes :
- les lamelles de fixation comportent chacune au moins une perforation longitudinale offrant une pluralité de positionnements par rapport aux renforts latéraux et/ou au bâti.
- les lamelles de fixation ont une forme coudée assurant un positionnement angulaire des renforts latéraux par rapport à l'assise ou au dossier.
- les perforations linéaires du bâti sont positionnées de façon à former deux lignes pointillées parallèles, de part et d'autre d'une longueur du bâti.
- la coque externe comporte au moins un élément de renfort amovible, fixé sur le bâti au moyen de lamelles perforées et d'éléments de fixation insérés dans les perforations dudit bâti, ces éléments de renfort venant compléter les renforts latéraux pour soutenir le patient.
- la coque interne en mousse est constituée d'une pluralité de blocs en mousse de densités différentes, lesdits blocs étant assemblés les uns avec les autres et maintenus par l'intermédiaire de la coque externe.

### Brève description des dessins

L'invention sera mieux comprise à la lecture de la description qui suit et à l'examen des figures qui l'accompagnent. Celles-ci sont présentées à titre indicatif et nullement limitatif de l'invention.
Les figures 1 et 2 représentent un corset orthopédique selon l'art antérieur.
La figure 3A représente une vue générale de face d'un corset orthopédique selon l'invention.
La figure 3B représente une vue générale de coté d'un corset orthopédique selon l'invention, avec sa coque externe et sa coque interne.
Les figures 4A et 4B représentent des vues respectivement de dos et de face d'un mode de réalisation du bâti d'une coque externe de corset orthopédique selon l'invention.
La figure 5 représente une vue de face d'un autre mode de réalisation du bâti d'une coque externe de corset orthopédique selon l'invention.
La figure 6 représente une vue de face d'un exemple de coque interne d'un corset orthopédique selon l'invention.

### Description détaillée de modes de réalisation de l'invention

L'invention concerne un corset orthopédique évolutif permettant de déplacer et/ou de moduler les différents éléments du corset en fonction des évolutions morphologiques et des besoins différentiels du patient.

Un exemple d'un tel corset orthopédique prêt à l'usage est montré sur la figure 3A. Ce corset orthopédique est un corset siège 100 réalisé sur mesures ou sur moulage pour le patient, en fonction de sa morphologie, de sa stature et de ses déficiences à corriger. Ce corset siège 100, appelé par la suite simplement corset, comporte un dossier 30, une assise 40 et des renforts latéraux 5 destinés à soutenir au moins un segment corporel du patient tel que le tronc ou un membre inférieur du patient. Ces renforts latéraux 50 peuvent comporter des renforts latéraux inférieurs 51 pour maintenir les membres inférieurs du patient et des renforts latéraux supérieurs 52, 53 pour maintenir le tronc du patient. Comme le montrent les renforts latéraux 53 de cette figure 3A, ces renforts latéraux peuvent avoir des formes et des dimensions différentes.

Le corset 100 peut comporter des éléments de renfort supplémentaires, tels qu'un renfort d'entrejambes 60 pour maintenir chacune des jambes d'un patient séparément, un appui-tête 70 pour soutenir la tête du patient, un renfort dorsal pour maintenir le dos du patient ou un repose-pied pour supporter ses pieds.

La figure 3B montre un corset selon l'invention en cours de montage. Ce corset 100 comporte une coque interne en mousse 110 destinée à épouser les contours morphologiques du patient et une coque externe 120 à l'intérieur de laquelle est montée la coque interne 110 et destinée à former une structure rigide de renfort autour de la coque en mousse 110. Comme montré sur la figure 3A, l'ensemble est revêtu d'une housse qui permet non seulement d'améliorer l'esthétique générale du corset mais aussi d'augmenter la résistance à l'usure de la coque en mousse, de faciliter l'entretien et d'augmenter l'hygiène dudit.

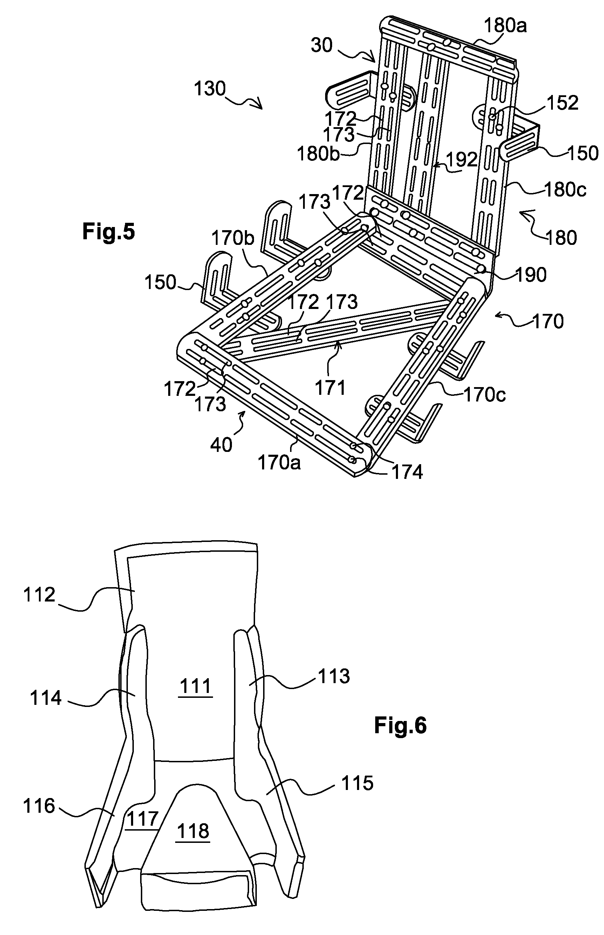

La coque en mousse 110 est réalisée, par exemple, par un moulage du tronc du patient au moyen, par exemple d'un relevé corporel 3D réalisé par scanner numérique. Le volume et les formes du patient sont alors reconstitués virtuellement. Ensuite, le moulage est travaillé pour apporter les corrections orthopédiques souhaitées afin de créer des zones de décharge d'appui et des zones d'appui correcteur ou de soutien dans la coque. Une fois le modèle achevé, une fraiseuse numérique (ou robot numérique) permet de creuser la forme du patient dans un bloc de mousse ou dans plusieurs blocs de mousse présentant des densités différentes. Ces blocs de densités différentes permettent d'adapter la coque interne aux impératifs de maintien orthopédique et de confort du patient. Un exemple de coque interne 110 est représenté sur la figure 6. Dans cet exemple, la coque interne 110 est réalisée à partir de plusieurs blocs en mousse, de densités différentes. L'assise 117 et le renfort d'entrejambes 118 sont réalisés dans un même bloc de mousse ; les renforts latéraux 113 et 115 sont réalisés dans un même bloc de mousse ; de même les renforts latéraux 114 et 116 sont réalisés dans un même bloc de mousse ; le dossier 111 et un renfort cervical 112 sont réalisés dans un même bloc de mousse, tous ces blocs de mousse étant indépendants les uns des autres et assemblés les uns avec les autres par l'intermédiaire de leurs fixations respectives sur la structure.

La coque interne 110 ainsi réalisée peut alors être introduite dans la coque externe 120. Cette coque externe 120 comporte un bâti 130 formant la structure rigide du dossier 30 et de l'assise 40 du corset 100. Elle comporte en outre des renforts latéraux 140 fixés sur le bâti au moyen de lamelles de fixation 150. Les renforts latéraux 140 sont des plaques de métal ou de plastique, placées sur les cotés du corset pour soutenir un membre ou un segment de membre d'un patient. Chaque renfort latéral est découpé à des dimensions et à la forme appropriées pour soutenir certaines zones de la coque interne en mousse.

Comme montré sur la figure 3B, des lamelles de fixation sont aptes à être fixées d'une part sur les renforts latéraux et d'autre part sur le bâti afin d'assembler les renforts latéraux 140 avec le bâti 130.

Chaque lamelle de fixation peut avoir une forme coudée, c'est-à-dire qu'elle peut comporter, dans sa partie centrale 150c, un angle arrondi 153 permettant un positionnement angulaire des renforts latéraux par rapport au bâti 130. L'angle 153 formé dans la zone centrale 150c de la lamelle de fixation peut avoir des mesures différentes en fonction des besoins orthopédiques du patient.

Chaque lamelle de fixation comporte au moins une perforation 151 permettant la traversée d'éléments de fixations 152 du type vis ou rivets. Ces perforations 152 peuvent être longitudinales de façon à offrir une pluralité de positionnements par rapport aux renforts latéraux et/ou au bâti. Dans un mode de réalisation de l'invention, chaque lamelle de fixation comporte une seule perforation longitudinale sur toute la longueur de la lamelle. Dans un autre mode de réalisation, montré sur la figure 3B, chaque lamelle comporte au moins deux perforations 151 a, 151b, une perforation 151 a étant réalisée sur l'extrémité 150a de la lamelle positionnée en regard du renfort latéral 140, l'autre perforation 151b étant réalisée sur l'extrémité 150b de la lamelle positionnée face au bâti 130.

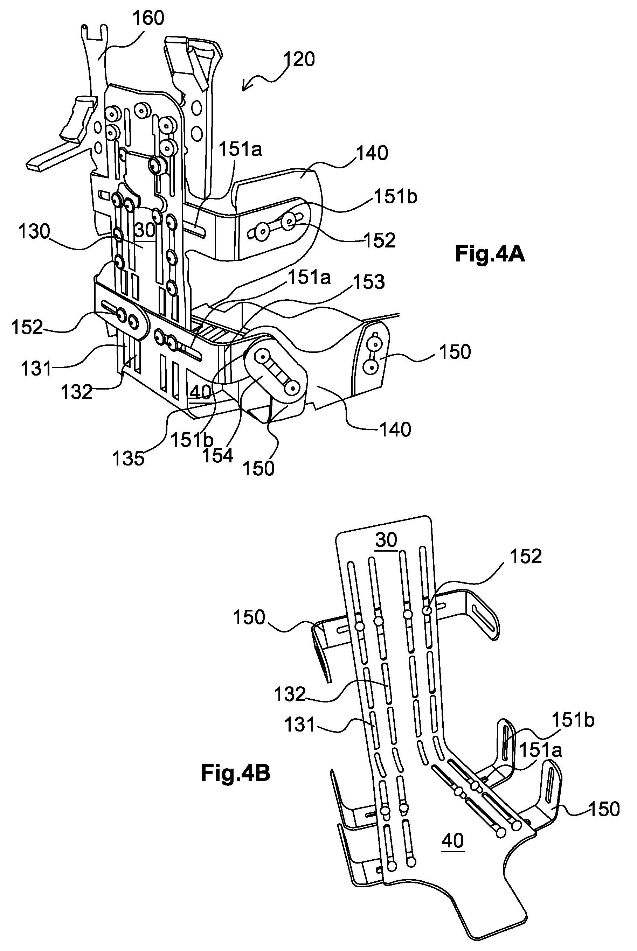

Un premier exemple d'une coque externe 120 est représenté sur les figures 4A et 4B. Dans un mode de réalisation de l'invention, la coque externe 120 comporte un bâti 130 constitué d'une unique plaque métallique pliée pour former, d'une part, l'assise 40 et, d'autre part, le dossier 30. La zone de pliure 135 de la plaque métallique formant le bâti 130 constitue une zone de jonction du dossier 30 et de l'assise 40. Cette zone de pliure 135 peut former un angle de plus ou moins 90° en fonctbn des besoins morphologiques du patient.

La plaque métallique, par exemple de l'aluminium, formant le bâti 130 comporte des perforations linéaires 131, 132 réparties sur toute sa longueur. Ces perforations linéaires 131, 132 forment des ouvertures oblongues situées parallèlement au contour de la plaque métallique. Dans une variante, ces perforations linéaires 131 et 132 sont positionnées de façon à former deux lignes pointillées parallèles de part et d'autre de la longueur du bâti. Cette double ligne des perforations permet d'ajuster très précisément la position des renforts latéraux par rapport à l'assise ou au dossier, via les lamelles de fixation 150. De plus, cette double ligne de perforations permet de fixer chaque renfort latéral 140 au moyen de deux éléments de fixation 152 afin de consolider l'assemblage entre le renfort latéral et le bâti.

Dans les exemples de coques externes montrées sur les figures 3, 4A et 4B, la plupart des lamelles de fixation 150 sont courbes. Toutefois, des lamelles de fixation planes 154 peuvent également être utilisées, par exemple pour fixer un appui tête 160 ou pour renforcer l'écart angulaire entre l'assise 40 et le dossier 30, comme montré sur la figure 4A. Dans ce cas, les lamelles de fixation planes peuvent être de petite longueur ; elles comportent alors une seule perforation longitudinale.

Un deuxième mode de réalisation d'une coque externe 120 est représenté schématiquement sur la figure 5. Dans ce mode de réalisation, le bâti 130 de la coque externe 120 comporte un premier cadre 170, ou cadre inférieur, formant l'assise 40 de la coque externe et un second cadre 180, ou cadre supérieur, formant le dossier 30 de ladite coque externe. Dans ce mode de réalisation, le cadre inférieur 170 et le cadre supérieur 180 sont assemblés l'un avec l'autre au moyen d'une plaque intermédiaire coudée 190, formant à la fois un coté du cadre inférieur et un coté du cadre supérieur, ces deux cotés étant séparés par une zone angulaire formant un coude 191.

Le cadre inférieur 170 comporte au moins trois lames perforées 170a, 170b, 170c. Ces lames perforées sont fixées les unes aux autres au moyen d'éléments de fixation 174 du type vis ou rivets. Elles sont en outre fixées à la plaque intermédiaire coudée 190 de façon à former un quadrilatère constituant l'assise de la coque externe. De même, le cadre supérieur 180 comporte au moins trois lames perforées 180a, 180b, 180c. Ces lames perforées sont fixées les unes aux autres au moyen d'éléments de fixation du type vis ou rivets. Elles sont en outre fixées à la plaque intermédiaire coudée 190 de façon à former un quadrilatère constituant le dossier de la coque externe.

Des lamelles de fixation 150, identiques à celles décrites dans le mode de réalisation précédent, sont fixées sur les cadres inférieur et/ou supérieur par l'intermédiaire d'éléments de fixation 152 pour assurer l'assemblage des renforts latéraux avec le bâti.

Chacune des lames perforées du cadre inférieur et du cadre supérieur comporte une pluralité de perforations linéaires 172, 173 réparties sur toute la longueur de ladite lame. De même, la plaque intermédiaire coudée 190 comporte une pluralité de perforations linéaires 172, 173 réparties sur toute sa longueur. Ces perforations linéaires 171, 172 forment des ouvertures oblongues destinées à recevoir les éléments de fixation 152. Dans une variante, ces perforations linéaires 172 et 173 sont positionnées de façon à former deux lignes pointillées parallèles sur tout le tour de chacun des cadres. La plaque intermédiaire coudée 190 comporte quatre lignes pointillées parallèles, deux lignes situées avant le coude 191 et deux lignes situées après le coude 191. Cette double ligne des perforations permet d'ajuster très précisément la position des renforts latéraux par rapport à l'assise ou au dossier, via les lamelles de fixation 150. De plus, cette double ligne de perforations permet de fixer chaque renfort latéral 140 au moyen de deux éléments de fixation 152 afin de renforcer l'assemblage renfort latéral / bâti.

Le cadre supérieur 180 comme le cadre inférieur 170 peuvent comporter une ou plusieurs lame(s) de renfort destinée(s) à renforcer l'assise 40 et/ou le dossier 30. Cette lame de renfort peut être positionnée dans la diagonale du dossier ou de l'assise, comme la lame 171 du cadre inférieur de la figure 5. Elle peut aussi être positionnée transversalement ou longitudinalement comme la lame 192 du cadre supérieur de la figure 5. Ces lames de renfort peuvent comporter également des perforations linéaires 172, 173 identiques aux perforations linéaires des lames formant les cadres.

Dans le mode de réalisation qui vient d'être décrit les lames perforées formant le cadre inférieur 170 et le cadre supérieur 180 peuvent être métalliques, par exemple en aluminium. Elles peuvent également être en totalité ou partiellement en plastique rigide. Un cadre peut ainsi être réalisé par un assemblage de lames perforées en métal et de lames perforées en plastique.

Le corset 100 qui vient d'être décrit présente l'avantage d'être totalement modulable et donc évolutif. La coque interne 110 peut être réajustée en fonction des évolutions des besoins ou de la morphologie du patient en retravaillant le ou les blocs de mousse par exemple en les creusant d'avantage, en les redécoupant, en rajoutant des morceaux de mousse, etc. La coque interne peut ainsi être modifiée jusqu'à s'ajuster exactement à la morphologie du patient et à la position qu'on souhaite lui voir maintenir. De plus, tous les éléments complétant le bâti étant amovibles et déplaçables par rapport audit bâti, la coque externe 120 peut être adaptée à la coque interne 110, quelles que soient les modifications de ladite coque interne. Des renforts latéraux, dorsaux ou autres éléments de soutien peuvent être ajoutés ou retirés en fonction des besoins du patient. Ainsi, la totalité du corset peut être modifiée aussi souvent que nécessaire en fonction des besoins du patient qui peuvent varier au cour du temps, pour des raisons médicales et/ou physiologiques.

## Revendications

1. Corset orthopédique (100) pour le soutien et le maintien dans une position donnée d'au moins un segment corporel d'un patient, ledit corset orthopédique comportant
- une coque interne (110) en mousse destinée à épouser des contours morphologiques du au moins un segment corporel du patient et comportant au moins un bloc de mousse creusé en fonction des contours morphologiques dudit segment corporel du patient à soutenir, et
- une coque externe (120) à l'intérieur de laquelle est montée la coque interne en mousse et formant une structure rigide de renfort,
comportant :
- un bâti (130) formant un dossier et une assise, muni de perforations linéaires,
- des renforts latéraux (140) amovibles, et
- des lamelles de fixation (150) perforées aptes à être fixées sur les renforts latéraux, d'une part, et sur le bâti, d'autre part, au moyen d'éléments de fixation (152) introduits dans les perforations linéaires du bâti et les perforations des lamelles de fixation,
- **caractérisé en ce que** le bâti (130) est formé :
- soit par une unique plaque métallique pliée pour former, d'une part, l'assise (40) et, d'autre part, le dossier (30) de la coque externe,
- soit par un premier cadre (170) formant l'assise de la coque externe, et par un second cadre (180) formant le dossier de ladite coque externe, une plaque intermédiaire coudée (190) étant fixée sur le premier et sur le second cadres et fermant lesdits premier et second cadres, chaque cadre (170, 180) étant formé d'au moins trois lames perforées (170a, 170b, 170c, 180a, 180b, 180c) fixées les unes aux autres et/ou à la plaque intermédiaire coudée (190) et aptes chacune à recevoir les éléments de fixation des lamelles de fixation.

2. Corset orthopédique selon la revendication 1, **caractérisé en ce que** les lamelles de fixation (140) comportent chacune au moins une perforation longitudinale (151) offrant une pluralité de positionnements par rapport aux renforts latéraux et/ou au bâti.

3. Corset orthopédique selon l'une quelconque des revendications 1 à 2, **caractérisé en ce que** les lamelles de fixation (140) ont une forme coudée assurant un positionnement angulaire des renforts latéraux par rapport à l'assise (40) ou au dossier (30).

4. Corset orthopédique selon l'une quelconque des revendications 1 à 3, **caractérisé en ce que** les perforations linéaires (131, 132 et 172, 173) du bâti sont positionnées de façon à former deux lignes pointillées parallèles, de part et d'autre d'une longueur du bâti.

5. Corset orthopédique selon l'une quelconque des revendications 1 à 4, **caractérisé en ce que** la coque externe (120) comporte au moins un élément de renfort amovible (160), fixé sur le bâti au moyen de lamelles perforées et d'éléments de fixation insérés dans les perforations dudit bâti.

6. Corset orthopédique selon l'une quelconque des revendications 1 à 5, **caractérisé en ce que** la coque interne (110) en mousse est constituée d'une pluralité de blocs en mousse de densités différentes, lesdits blocs étant assemblés les uns avec les autres et maintenus par l'intermédiaire de la coque externe.

## Patentansprüche

1. Orthopädisches Korsett (100) zum Stützen und zum Halt in einer gegebenen Stellung mindestens eines Körpersegments eines Patienten, wobei das orthopädische Korsett aufweist
- eine Innenschale (110) aus Schaumstoff, die dazu bestimmt ist, sich an morphologische Umrisse des mindestens einen Körpersegments des Patienten anzupassen und mindestens einen Schaumstoffblock aufweist, der abhängig von den morphologischen Umrissen des zu stützenden Körpersegments des Patienten ausgehöhlt ist, und
- eine Außenschale (120), in deren Inneres die Innenschale aus Schaumstoff montiert ist und die eine steife Verstärkungsstruktur bildet,
das aufweist:
- ein eine Rückenlehne und eine Sitzfläche bildendes Gestell (130), das mit linearen Durchbohrungen versehen ist,
- entfernbare seitliche Verstärkungen (140), und
- durchbohrte Befestigungslamellen (150), die an den seitlichen Verstärkungen einerseits und am Gestell andererseits mittels Befestigungselementen (152) befestigt werden können, die in die linearen Durchbohrungen des Gestells und die Durchbohrungen der Befestigungslamellen eingeführt werden,
**dadurch gekennzeichnet, dass** das Gestell (130) gebildet wird durch:
- entweder eine einzige Metallplatte, die gebogen ist, um einerseits die Sitzfläche (40) und andererseits die Rückenlehne (30) der Außenschale zu formen,
- oder einen ersten Rahmen (170), der die Sitzfläche der Außenschale bildet, und einen zweiten Rahmen (180), der die Rückenlehne der Außenschale bildet, wobei eine umgebogene Zwischenplatte (190) am ersten und am zweiten Rahmen befestigt ist und den ersten und den zweiten Rahmen schließt, wobei jeder Rahmen (170, 180) von mindestens drei durchbohrten Lamellen (170a, 170b, 170c, 180a, 180b, 180c) gebildet wird, die aneinander und/oder an der umgebogenen Zwischenplatte (190) befestigt und je fähig sind, die Befestigungselemente der Befestigungslamellen aufzunehmen.

2. Orthopädisches Korsett nach Anspruch 1, **dadurch gekennzeichnet, dass** die Befestigungslamellen (140) je mindestens eine Längsdurchbohrung (151) aufweisen, die eine Vielzahl von Positionierungen bezüglich der seitlichen Verstärkungen und/oder des Gestells anbietet.

3. Orthopädisches Korsett nach einem der Ansprüche 1 bis 2, **dadurch gekennzeichnet, dass** die Befestigungslamellen (140) eine umgebogene Form haben, die eine Winkelpositionierung der seitlichen Verstärkungen bezüglich der Sitzfläche (40) oder der Rückenlehne (30) gewährleistet.

4. Orthopädisches Korsett nach einem der Ansprüche 1 bis 3, **dadurch gekennzeichnet, dass** die linearen Durchbohrungen (131, 132 und 172, 173) des Gestells so positioniert sind, dass sie zwei parallele gestrichelte Linien zu beiden Seiten einer Länge des Gestells bilden.

5. Orthopädisches Korsett nach einem der Ansprüche 1 bis 4, **dadurch gekennzeichnet, dass** die Außenschale (120) mindestens ein entfernbares Komfortelement (160) aufweist, das am Gestell mittels durchbohrter Lamellen und Befestigungselementen befestigt ist, die in die Durchbohrungen des Gestells eingeführt werden.

6. Orthopädisches Korsett nach einem der Ansprüche 1 bis 5, **dadurch gekennzeichnet, dass** die Innenschale (110) aus Schaumstoff aus einer Vielzahl von Schaumstoffblöcken unterschiedlicher Dichten besteht, wobei die Blöcke zusammengebaut und mittels der Außenschale gehalten werden.

## Claims

1. Orthopaedic support (100) for supporting at least one section of a patient's body and holding it in a given position, the said orthopaedic support comprising
- an internal shell (110) made of foam intended to hug the morphological contours of the at least one section of the patient's body and comprising at least one block of foam that is hollowed to suit the morphological contours of the said section of the patient's body that is to be supported, and
- an external shell (120) in which the foam internal shell is mounted and that forms a rigid reinforcing structure,
comprising:
- a framework (130) forming a backrest and a sitting part, provided with linear perforations,
- removable lateral reinforcers (140), and
- perforated fixing straps (150) that can be fixed to the lateral reinforcers, on the one hand, and to the framework, on the other, by means of fasteners (152) introduced in the linear perforations of the framework and the perforations in the fixing straps,
**characterized in that** the framework (130) is formed:
- either of a single metal sheet that is bent to form, on the one hand, the sitting part (40) and, on the other hand, the backrest (30) of the external shell,
- or by a first frame (170) that forms the sitting part of the external shell and by a second frame (180) that forms the backrest of the said external shell, a bent intermediate plate (190) being fixed to the first and second frames and closing the said first and second frames, each frame (170, 180) being formed of at least three perforated strips (170a, 170b, 170c, 180a, 180b, 180c) fixed together and/or to the bent intermediate plate (190) and each able to accept the fasteners of the fixing straps.

2. Orthopaedic support according to Claim 1, **characterized in that** the fixing straps (140) each comprise at least one longitudinal perforation (151) offering a plurality of positionings with respect to the lateral reinforcers and/or to the framework.

3. Orthopaedic support according to either one of Claims 1 and 2, **characterized in that** the fixing straps (140) have a bent shape providing angular positioning of the lateral reinforcers with respect to the sitting part (40) or to the backrest (30).

4. Orthopaedic support according to any one of Claims 1 to 3, **characterized in that** the linear perforations (131, 132 and 172, 173) of the framework are positioned in such a way as to form two parallel broken lines, on either side of a length of the framework.

5. Orthopaedic support according to any one of Claims 1 to 4, **characterized in that** the external shell (120) comprises at least one removable reinforcing element (160) that is fixed to the framework by means of perforated straps and of fasteners inserted into the perforations of the said framework.

6. Orthopaedic support according to any one of Claims 1 to 5, **characterized in that** the internal shell (110) made of foam is made up of a plurality of blocks of foam of different densities, the said blocks being assembled with one another and held in position by the external shell.
